Europäisches Patentamt

European Patent Office

Office européen des brevets

(11) Veröffentlichungsnummer: **0 361 137**
**A1**

## EUROPÄISCHE PATENTANMELDUNG

(21) Anmeldenummer: **89116322.2**

(22) Anmeldetag: **04.09.89**

(51) Int. Cl.5 **G01R 33/035**

(30) Priorität: **16.09.88 DE 3831545**

(43) Veröffentlichungstag der Anmeldung:
**04.04.90 Patentblatt 90/14**

(84) Benannte Vertragsstaaten:
**DE FR GB IT NL**

(71) Anmelder: **Siemens Aktiengesellschaft**
**Wittelsbacherplatz 2**
**D-8000 München 2(DE)**

(72) Erfinder: **Hoenig, Eckhardt, Dr.**
**Palmstrasse 1a**
**D-8520 Erlangen(DE)**

(54) **Magnetometer-Einrichtung mit einem Dewar-Gefäss zur Messung schwacher Magnetfelder.**

(57) Die Magnetometer-Einrichtung zur Messung schwacher, von mindestens einer zu detektierenden Feldquelle hervorgerufener Magnetfelder enthält ein Dewar-Gefäß (2), das einen Innenraum (3) mit einem Zugang über einen Hals (4) aufweist und in dessen Innenraum mehrere supraleitende Gradiometer (24a-24c) sowie ihnen zugeordnete SQUIDs (Chip 21) angeordnet sind. Das Dewar-Gefäß (2) soll unterhalb oder seitlich von der Feldquelle angeordnet sein. In seinem Innenraum (3) soll sich ein Einsatzbehälter (18) für ein flüssiges Kältemittel ($K_1$) befinden, das thermisch mit den SQUIDs (21) verbunden ist. Dem Einsatzbehälter (18) entnommenes gasförmiges Kältemittel ($K_2$) ist über schlauchartige Leitungen (25) den Gradiometern (24a-24c) zuzuführen. Das an den Gradiometern (24a-24c) in den Innenraum (3) entweichende gasförmige Kältemittel ($K_3$) ist über den Hals (4) nach außen abzuführen. In den Hals (4) ist eine einen Rückstrom von wärmerem gasförmigen Kühlmittel in den Innenraum (3) unterbindende Drosselvorrichtung (27) eingesetzt.

FIG 2

# Magnetometer-Einrichtung mit einem Dewar-Gefäß zur Messung schwacher Magnetfelder

Die Erfindung bezieht sich auf eine Magnetometer-Einrichtung zur Messung schwacher, von mindestens einer zu detektierenden Feldquelle hervorgerufener Magnetfeld mit einem Dewar-Gefäß, das einen Innenraum mit einem Zugang über einen Hals aufweist und in dessen Innenraum mehrere supraleitende Gradiometer sowie ihnen zugeordnete SQUIDs angeordnet sind, die von einem von außen über den Hals zugeführten Kältemittel gekühlt sind. Eine derartige Magnetometer-Einrichtung ist z.B. in der Veröffentlichung "Physics Today", März 1986, Seiten 36 bis 44 angedeutet.

Zur Messung sehr schwacher Magnetfelder ist eine Verwendung von supraleitenden Quanten-Interferometern, sogenannten "SQUIDs" (Abkürzung von: "Superconducting QUantum Interference Devices"), allgemein bekannt (vgl. z.B. "J.Phys.E.: Sci.Instrum.". Vol. 13, 1980, Seiten 801 bis 813 oder "IEEE Trans. Electron Dev.", Vol. ED-27, No. 10, Oktober 1980, Seiten 1896 bis 1908). Als ein bevorzugtes Anwendungsgebiet für diese Interferometer wird auf dem Gebiet der medizinischen Diagnostik die Magnetokardiographie oder Magnetoenzephalographie angesehen. Die hierbei hervorgerufenen Magnetfelder von magnetischen Herz- bzw. Gehirnwellen haben nämlich Feldstärken in der Größenordnung von nur etwa 50 pT bzw. 0,1 pT (vgl. z.B. "Biomagnetism - Proceedings Third international Workshop on Biomagnetism, Berlin 1980", Berlin, New York 1981, Seiten 3 bis 31). Diese sehr schwachen Felder müssen zudem noch bei Anwesenheit von verhältnismäßig großen Störfeldern zu detektieren sein.

Zur Messung derartiger biomagnetischer Felder in der genannten Größenordnung sind Meßeinrichtungen bekannt, die ein- oder insbesondere auch mehrkanalig ausgeführt sein können (vgl. z.B. DE-OS 32 47 543). Diese Einrichtungen enthalten je nach Anzahl der Kanäle SQUID-Magnetometer mit Gradiometern 1. oder höherer Ordnung.

Eine entsprechende Magnetometer-Einrichtung ist aus der eingangs genannten Literaturstelle "Physics Today" ersichtlich. Die supraleitenden Gradiometer sind zusammen mit den ihnen zugeordneten SQUIDs innerhalb eines Dewar-Gefäßes angeordnet. Hierbei ist eine Kühlung der supraleitenden Einbauten, insbesondere der hochempfindlichen SQUIDs, unter Vermeidung von direkten und indirekten magnetischen Störungen erforderlich. Man verzichtet deshalb auf eine Kühltechnik unter Einsatz von Kältemaschinen, die mit ihrer elektromagnetischen Abstrahlung und mit ihren bewegten magnetischen Teilen über Vibrationen magnetische Störfelder erzeugen. Gekühlt wird folglich mit einem Vorrat an Kältemittel, insbesondere flüssigem Helium, der in Zeitabständen von mehreren Tagen zu ergänzen ist.

Bei der bekannten Magnetometer-Einrichtung sind jedoch die die supraleitenden Gradiometer und SQUIDs aufnehmenden Dewar-Gefäße oberhalb der zu detektierenden Feldquelle eines zu untersuchenden Patienten angeordnet. Geht man von Füllmengen von etwa 15 bis 30 l an flüssigem Kältemittel pro Dewar aus, wie sie z.B. für mehrkanalige Einrichtungen üblich sind, so führt die gewählte Anordnung der Dewar-Gefäße zu einer Gefährdung des direkt darunterliegenden Patienten. Im Fall eines plötzlichen Zusammenbruchs des Isoliervakuums bzw. einer plötzlichen Beschädigung eines Dewar-Gefäßes mit Ansprechen von Sicherheitsventilen würde sich nämlich eine nicht unbedenkliche Kaltgasmenge über den Patienten ergießen. So erzeugen z.B. die erheblichen Helium-Füllmengen von 15 bis 30 l Flüssigkeit in einem entsprechenden Notfall etwa 10 bis 30 m³ Helium-Kaltgas. Dieses Kaltgas ist schwerlich vom Patienten fernzuhalten, insbesondere dann, wenn die Untersuchung in einer Abschirmkabine durchgeführt wird. Eine Überkopf-Anordnung von Dewar-Gefäßen kann außerdem von Patienten als unangenehm empfunden werden.

Aufgabe der vorliegenden Erfindung ist es deshalb, die Magnetometer-Einrichtung der eingangs genannten Art dahingehend auszugestalten, daß die Anordnung mindestens eines Dewar-Gefäßes zu keiner Gefährdung eines Patienten führen kann und daß dennoch ein Einsatz von magnetische Störungen hervorrufenden Kältemaschinen nicht erforderlich wird.

Diese Aufgabe wird erfindungsgemäß dadurch gelöst,

- daß das Dewar-Gefäß unterhalb oder seitlich von der mindestens einen Feldquelle angeordnet ist,

- daß in dem Innenraum ein besonderer Einsatzbehälter vorgesehen ist, in den über eine durch den Hals führende Rohrleitung flüssiges Kältemittel einzuspeisen ist,

- daß sich in oder an dem Einsatzbehälter die SQUIDs befinden,

- daß dem Einsatzbehälter entnommenes gasförmiges Kältemittel über mindestens eine rohrförmige Verbindungsleitung den supraleitenden Gradiometern zuzuführen ist,

- daß das an den Gradiometern in den Innenraum entweichende gasförmige Kältemittel über den Hals nach außen abzuführen ist
und

- daß in den Hals eine einen Rückstrom von wärmerem gasförmigen Kühlmittel in den Innenraum unterbindende Drosselvorrichtung eingesetzt ist.

Die mit dieser Ausgestaltung der Erfindung verbundenen Vor teile sind insbesondere darin zu sehen, daß eine Überkopf-Anordnung des Dewar-Gefäßes vermieden wird und dennoch eine effektive Kühlung der supraleitenden Teile der Einrichtung zu gewährleisten ist. Hierbei werden unerwünschte Konvektionen von Abgas des Kältemittels unterbunden. So sorgen z.B. die rohrförmigen Verbindungsleitungen für eine ungestörte Zufuhr von besonders kaltem Abgas zu den supraleitenden Gradiometern, obwohl diese Gradiometer im allgemeinen geodätisch höher als der Einsatzbehälter und somit entgegengesetzt zu der Richtung angeordnet sind, in der das Kältemittel aufgrund einer durch Temperatur- und Dichteunterschiede angefachten Konvektion zu strömen versucht. Die im Bereich des Halses angeordnete Drosselvorrichtung verhindert außerdem, daß wärmeres Abgas von außen in den Innenraum zurückfließen kann.

Vorteilhafte Ausgestaltungen der Magnetometer-Einrichtung nach der Erfindung gehen aus den Unteransprüchen hervor.

Zur weiteren Erläuterung der Erfindung wird nachfolgend auf die schematische Zeichnung Bezug genommen, in deren Figur 1 eine Magnetometer-Einrichtung in einer Patientenliege angedeutet ist. Figur 2 zeigt ein Dewar-Gefäß dieser Einrichtung. In den Figuren 3 und 4 ist eine Drosselvorrichtung in diesem Dewar-Gefäß in verschiedener Ansicht veranschaulicht. Dabei sind in den Figuren sich entsprechende Teile mit denselben Bezugszeichen versehen.

SQUID-Magnetometer, wie sie für ein- oder insbesondere mehrkanalige Einrichtungen nach der Erfindung vorgesehen werden können, sind prinzipiell bekannt. Sie enthalten zur Detektion der von einer Feldquelle, insbesondere vom Herz oder vom Gehirn eines Patienten ausgehenden magnetischen Signale (magnetische Flüsse oder Flußgradienten) mindestens ein supraleitendes Gradiometer. Die detektierten Signale werden dann über supraleitende oder normalleitende Verbindungsleiter einer der Kanalzahl entsprechenden Anzahl von SQUIDs zugeführt. Diesen SQUIDs, die ebenfalls zu einem Array zusammengefaßt sein können, ist eine Elektronik nachgeschaltet. In den Figuren sind diese bekannten Teile nicht näher ausgeführt.

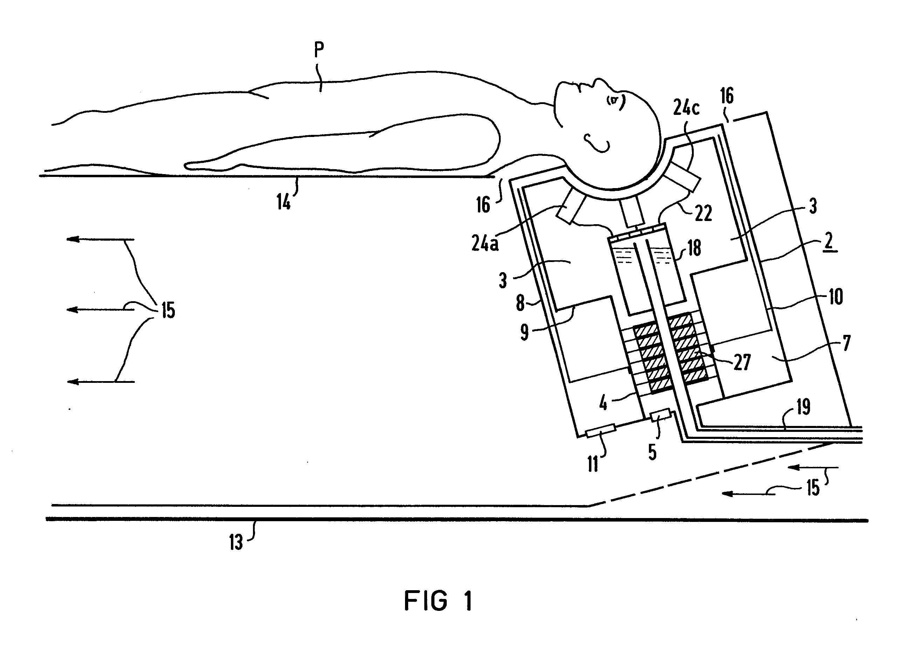

Gemäß dem in Figur 1 gezeigten Längsschnitt ist ein die supraleitenden Magnetometer aufnehmendes doppelwandiges Dewar-Gefäß 2 mit zumindest weitgehend rotationssymmetrischem Aufbau vorgesehen. Der Innenraum 3 dieses Gefäßes erlaubt einen Zugang über einen verhältnismäßig engen Hals 4. Er ist außerdem im Halsbereich mit mindestens einem Sicherheitsventil 5 versehen, über das in einem Störungsfall Kaltgas nach außen entweichen kann. Der Zwischenraum 7 zwischen den Wänden 8 und 9 des Gefäßes 2 ist in bekannter Weise evakuiert und enthält gegebenenfalls eine in der Figur nicht dargestellte Superisolation. Außerdem kann in ihm ein thermischer Strahlungsschild 10 angeordnet sein. Für den Vakuumraum 7 ist im Halsbereich ebenfalls mindestens ein Sicherheitsventil 11 vorgesehen.

Das Dewar-Gefäß 2 ist vorteilhaft so angeordnet, daß bei zumindest weitgehend rotationssymmetrischer Ausgestaltung sein Hals 4 geodätisch tiefer liegt als sein Innenraum 3. Gemäß dem dargestellten Ausführungsbeispiel ist der Hals 4 schräg nach unten geneigt. Diese Anordnungsart ermöglicht es, daß das Gefäß 2 in eine auf einem Boden 13 stehende Patientenliege 14 integriert werden kann. Gemäß dem dargestellten Ausführungsbeispiel ist das Dewar-Gefäß 2 dabei so verkippt, daß sich eine bequeme Liegeposition eines Patienten P ergibt. Von diesem Patienten soll beispielsweise das magnetische Gehirnfeld untersucht werden. Aus diesem Grunde ist die Form des Dewar-Gefäßes der Kopfform des Patienten entsprechend angepaßt.

Der Unterbau der Patientenliege 14 wird vorteilhaft auf vollem Querschnitt zur passiven und/oder zuzüglich aktiven Unter druck-Lüftung des Dewar-Bereiches genutzt. Die entsprechenden Strömungsverhältnisse einer aktiven Durchgangs-Lüftung sind in der Figur durch gepfeilte Linien 15 angedeutet. Zusätzlich kann in der Patientenliege 14 ein Absaugspalt 16 um das Dewar-Gefäß 2 herum vorgesehen werden. Beim Ansprechen der Sicherheitsventile 5 oder 11 vom Innenraum 3 bzw. vom Vakuumraum 7 entweicht dann das entsprechende Kaltgas in den Unterbau der Patientenliege und wird dort auf großem Querschnitt abgesaugt.

Die Anordnung des Dewar-Gefäßes 2 unter dem Patienten P hat außerdem den Vorteil, daß große Massen zur Begrenzung von Vibrationsamplituden, die indirekt zu magnetischen Störungen führen könnten, unterhalb des Patienten anzubringen sind.

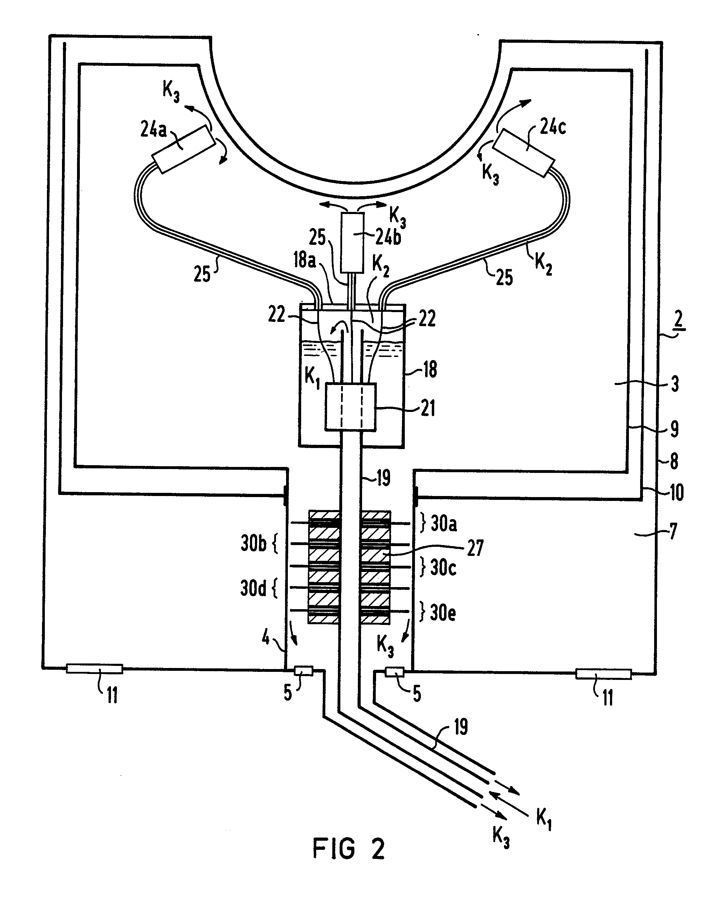

In Figur 2 ist das Dewar-Gefäß 2 als Längsschnitt näher veranschaulicht. In seinen Innenraum 3 ist ein Einsatzbehälter 18 eingebracht, der die für eine Arbeitsperiode, beispielsweise einen Arbeitstag, erforderliche Menge eines flüssigen Kältemittels $K_1$, z.B. 2 bis 3 l flüssiges Helium, aufnimmt. Dieses Kältemittel soll zur Kühlung der supraleitenden Teile der erfindungsgemäßen Einrichtung unmittelbar oder mittelbar dienen. Nachgefüllt wird das Kältemittel von unten über eine durch den Hals 4 führende Rohrleitung 19, welche eine vakuumisolierte und im Gegenstrom mit Abgas des Kältemittels gekühlte Verbindung zu einem externen, nicht dargestellten Vorratsgefäß darstellt. Die Nachfüllung kann dabei repetierend, z.B. im Tagesrhythmus erfolgen. Im bzw. an dem Einsatzbehälter 18 sind die besonders temperaturempfindlichen Teile, insbesondere die SQUIDs angeordnet. Diese in der Figur nicht dargestellten SQUIDs sind beispielsweise zu einem Array zusammengefaßt, das sich auf einem nur angedeuteten Chip 21 befindet. Die SQUIDs sind über beispielsweise supraleitende Leiter 22 mit den entsprechenden Gradiometern verbunden. Von diesen Gradiometern sind in der Figur nur drei angedeutet und mit 24a bis 24c bezeichnet. Ihre Anzahl kann jedoch wesentlich darüber liegen. Die elektrisch leitenden Verbindungsleiter 22 sind innerhalb von schlauchartigen Kältemittelleitungen 25 angeordnet, die beispielsweise aus einem Kunststoff bestehen. Es können auch elektrische Verbindungsleiter 22 vorgesehen werden, die in die jeweils zugeordneten schlauchartigen Kältemittelleitungen integriert sind. So lassen sich z.B. bekannte folienartige Leiter auf mindestens einer ihrer Flachseiten der Folie mit einer schlauchartigen Hülle überziehen, beispielsweise bekleben, so daß dann die Leiter als Teil einer schlauchartigen Kältemittelleitung 25 anzusehen sind. Über die Leitungen 25 wird in dem Einsatzbehälter 18 entstehendes, verhältnismäßig kaltes gasförmiges Kältemittel $K_2$ den Gradiometern 24a bis 24c zugeführt. Der Einatzbehälter 18 ist hierzu mit einem Deckel 18a versehen, der als Gasverteiler ausgebildet ist und an den die Kältemittelleitungen 25 angeschlossen sind. Bei Bedarf wird die Abgasrate durch eine entsprechende Wärmezufuhr in den Einsatzbehälter 18, z.B. mittels eines wärmeleitenden Stabes aus glasfaserverstärktem Material, eingestellt. An den Gradiometern tritt das Kaltgas in den Innenraum 3 ein. Es verläßt dann diesen Innenraum an dem Hals 4. Dabei ist besondere Sorgfalt aufzuwenden, um einen Rückstrom von wärmerem Abgas in den Innenraum 3 aufgrund einer auf Temperatur- und Dichteunterschieden basierenden Konvektion zu verhindern. Zu diesem Zweck ist in dem Hals 4 eine Drosselvorrichtung 27 angeordnet, die nur einen verhältnismäßig engen Spalt für das Abgas $K_3$ freiläßt oder freigibt. Der mindestens eine Spalt bewirkt dabei, daß das in dem Innenraum unter Überdruck stehende Abgas $K_3$ nur nach außen tritt. Dieses Abgas kann gegebenenfalls dann noch zur Abkühlung von weiteren Teilen der Einrichtung wie z.B. des Strahlungsschildes 10 herangezogen werden. Zur weiteren Begrenzung einer möglichen Kaltgaskonvektion kann außerdem der Innenraum 3 gegebenenfalls noch mit einer Glaskugelfüllung versehen werden.

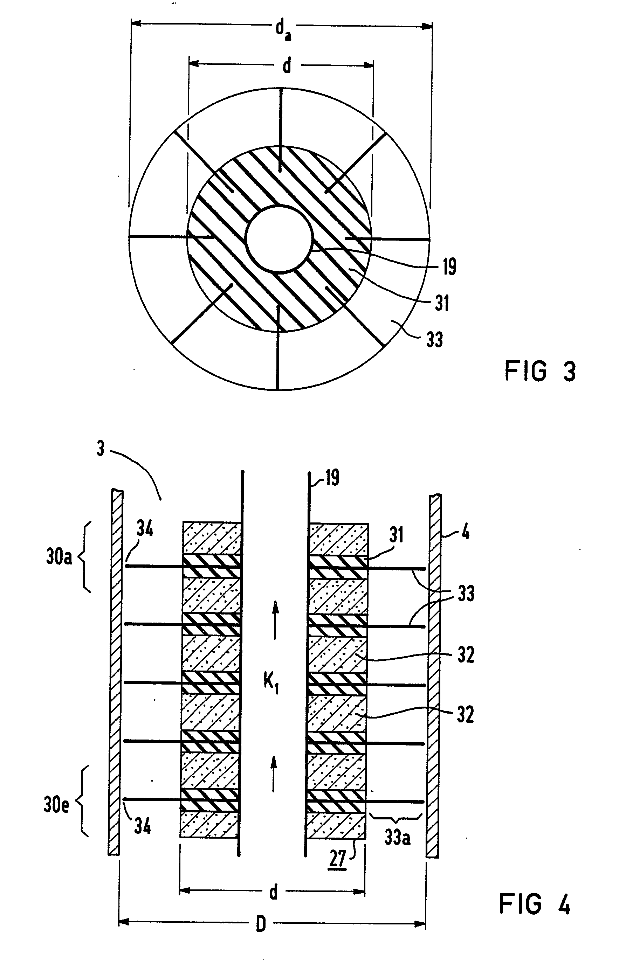

Eine Ausführungsform der Drosselvorrichtung 27 ist in den Figuren 3 und 4 als Querschnitt bzw. Längsschnitt schematisch veranschaulicht. Sie ist kreisringzylindrisch ausgebildet, wobei sie die Rohrleitung 19 für das flüssige Kältemittel $K_1$ umschließt. Beispielsweise ist die Vorrichtung auf die Rohrleitung aufgeschoben. Diese Vorrichtung 27 enthält einen Stapel aus mehreren, beispielsweise fünf kreiszylindrischen Elementen 30a bis 30e. Jedes dieser Drosselelemente weist dabei eine zentrale ringscheibenförmige Klemmhalterung 31 auf, die beispielsweise aus einem glasfaserverstärkten Kunststoff besteht. Der Außendurchmesser d der Halterung ist dabei deutlich geringer als der Innendurchmesser D des rohrförmigen Halses des Dewar-Gefäßes gewählt. Auf den gegenüberliegenden Flachseiten der einzelnen Halterungen befinden sich kreisringzylindrische Stopfen 32 mit entsprechendem Durchmesser d. Diese Stopfen, die beispielsweise aus einem Hartschaum bestehen können, dienen insbesondere zur Beabstandung der einzelnen Halterungen 31 untereinander. Von jeder dieser Halterungen wird ein ringscheibenförmiges Kunststoffplättchen 33 getragen, das insbesondere aus einer speziellen Polyesterfolie bestehen kann. Diese verhältnismäßig dünnen Plättchen 33 haben einen Außendurchmesser $d_a$, der nur geringfugig kleiner als der Innendurchmesser D des Halses 4 ist. Es verbleibt so zwischen der Innenwand des Halses 4 und den scheibenförmigen Kunststoffplättchen 33 jeweils ein geringer Spalt 34, durch den das Abgas $K_3$ aus dem Innenraum 3 des Dewar-Gefäßes nach außen tritt. Der ringscheibenförmige, über den Außenrand der Halterung 31 seitlich hinausragende Außenrandteil 33a jedes dünnen Plättchens 33 ist verhältnismäßig flexibel, so daß sich dieser Teil bei einem stärkeren Überdruck in dem Innenraum 3, beispielsweise in einem Störungsfall, verbiegen kann und so vorteilhaft zu einem entsprechend größeren Strömungsquerschnitt in den Spalten 34 für das Abgas $K_3$ führt. Gegebenenfalls ist es sogar möglich, daß die elastischen Außenrandteile 33a der Plättchen 33 bis an die Innenwand des Halses 4 heranragen und daß die Spalte 34 erst aufgrund eines Verbiegens der Teile 33a durch das unter Überdruck stehende Abgas $K_3$ hervorgerufen werden.

Gemäß dem in den Figuren dargestellten Ausführungsbeispiel wurde davon ausgegangen, daß das Dewar-Gefäß 2 eine bauchige Erweiterung seines Innenraumes 3 gegenüber dem Halsbereich aufweist (vgl. z.B. EP-A-0 200 958). In diesem Fall kann mit Halteelementen, die sich nach Einführung in den Innenraum aufspreizen, und/oder mit einer Steckverbindung zwischen dem Einsatzbehälter 18 und den vorher eingebauten Gradiometern 24a bis 24c gearbeitet werden. Selbstverständlich können auch Dewar-Gefäße ohne bauchige Erweiterungen vorgesehen sein, die keine besonderen Probleme beim Einbau der vorgesehenen Systeme bereiten. Bei beiden Gefäßformen sind die folgenden zwei Fälle zu unterscheiden:

1) Die SQUIDs sind mit den zugeordneten Gradiometern zu Modulen integriert. Hierbei müssen lediglich normalleitende Anschlüsse und die Abgaskühlverbindungen hergestellt werden.

2) Gemäß dem angenommenen Ausführungsbeispiel sind die SQUIDs im oder am Einsatzbehälter zusammengefaßt. Dann sind supraleitende Verbindungen zu den Gradiometern sowie die Abgaskühlungen der Gradiometer herzustellen.

In beiden Fällen wird der Deckel 18a des Einsatzbehälters 18 vorteilhaft als Gasverteiler ausgebildet und zusammen mit den Gradiometern vor Einbringen des Einsatzbehälters eingebaut. Im ersteren Fall wird vorteilhaft ein galvanischer Vielfachstecker (vgl. die genannte EP-A-0 200 958) vorgesehen. Demgegenüber ist im zweiten Fall vorteilhaft ein planerer Vielfach-Koppeltransformator zu realisieren, wobei die Verkopplung beim Einfügen des Einsatzbehälters von selbst erfolgt.

Ferner ist es für die erfindungsgemäße Einrichtung nicht unbedingt erforderlich, daß der Einsatzbehälter 18 und das ihn um gebende Dewar-Gefäß 2 dieselbe Achse aufweisen. Wird der Einsatzbehälter um einen vorgegebenen Winkel relativ zu der Dewar-Achse gedreht, so ergibt sich eine frei vorzugebende Standardlage der Dewar-Achse. Dann ist z.B. eine horizontalliegende Dewar-Achse möglich, die für einen Patienten bequemen Zugang zu dessen Scheitelregion ermöglicht, falls der Patient normal auf einer Patientenliege ruht. Hierbei muß jedoch stets gewährleistet bleiben, daß der Einsatzbehälter so angeordnet ist, daß an seinen Deckel lediglich das über dem flüssigen Kältemittel $K_1$ befindliche gasförmige Kältemittel $K_2$ gelangt. Bei der erfindungsgemäßen Einrichtung kann also bei insbesondere rotationssymmetrischem Aufbau der Hals 4 (schräg) nach unten bis horizontal geneigt sein. Prinzipiell spielt die Halsneigung keine besondere Rolle; jedoch muß die erwähnte Lage der Phasengrenze in dem Einsatzbehälter gegeben sein. Aus Gründen einer einfachen Montage und Halterung ist aber ein rotationssymmetrischer Aufbau mit beispielsweise schräg nach unten weisendem Hals (vgl. Fig. 1) von Vorteil.

**Ansprüche**

1. Magnetometer-Einrichtung zur Messung schwacher, von mindestens einer zu detektierenden Feldquelle hervorgerufener Magnetfelder mit einem Dewar-Gefäß, das einen Innenraum mit einem Zugang über einen Hals aufweist und in dessen Innenraum mehrere supraleitende Gradiometer sowie ihnen zugeordnete SQUIDs angeordnet sind, die von einem von außen über den Hals zugeführten Kältemittel gekühlt sind, **dadurch gekennzeichnet,**

-daß das Dewar-Gefäß (2) unterhalb oder seitlich von der mindestens einen Feldquelle angeordnet ist,

-daß in dem Innenraum (3) ein besonderer Einsatzbehälter (18) vorgesehen ist, in den über eine durch den Hals (4) führende Rohrleitung (19) flüssiges Kältemittel ($K_1$) einzuspeisen ist,

- daß sich in oder an dem Einsatzbehälter (18) die SQUIDs (Chip 21) befinden,

-daß dem Einsatzbehälter (18) entnommenes gasförmiges Kältemittel ($K_2$) über mindestens eine schlauchartige Leitung (25) den supraleitenden Gradiometern (24a-24c) zuzuführen ist,

- daß das an den Gradiometern (24a-24c) in den Innenraum (3) entweichende gasförmige Kältemittel ($K_3$) über den Hals (4) nach außen abzuführen ist und

- daß in den Hals (4) eine einen Rückstrom von wärmerem gasförmigen Kühlmittel in den Innenraum (3) unterbindende Drosselvorrichtung (27) eingesetzt ist.

2. Einrichtung nach Anspruch 1, **dadurch gekennzeichnet,** daß die Drosselvorrichtung (27) eine den Strömungswiderstand für das gasförmige Kältemittel ($K_3$) erhöhender Einsatz in dem Hals (4) ist, der aus mehreren, zu einem Stapel zusammengesetzten ringscheibenförmigen Drosselelementen (30a bis 30e) besteht, die zusammen mit der Innenwand des Halses (4) mindestens einen engen Spalt (34) für das gasförmige Kältemittel ($K_3$) ausbilden.

3. Einrichtung nach Anspruch 2, **dadurch gekennzeichnet,** daß die Drosselelemente (30a bis 30e) jeweils zumindest im Bereich ihrer ringscheibenförmigen Außenrandteile (33a) elastisch ausgebildet sind und daß der Spalt (34) aufgrund eines Verbiegens dieser ringscheibenförmigen Außenrandteile (33a) durch das gasförmige Kältemittel ($K_3$) hervorgerufen oder vergrößert ist.

4. Einrichtung nach Anspruch 2 oder 3, **dadurch gekennzeichnet,** daß jedes Drosselelement (30a -30e) eine zentrale, die Rohrleitung (19) für das Kältemittel ($K_1$) umschließende Klemmhalterung (31) enthält, an der ein ringscheibenförmiges Kunststoffplättchen (33) befestigt ist.

5. Einrichtung nach Anspruch 4, **dadurch gekennzeichnet,** daß der Außendurchmesser ($d_a$) des Kunststoffplättchens (33) wesentlich größer als der Außendurchmesser (d) der zentralen Klemmmhalterung (31) ist.

6. Einrichtung nach einem der Ansprüche 3 bis 5, **dadurch gekennzeichnet,** daß das Kunststoffplättchen (33) aus einer Polyesterfolie hergestellt ist.

7. Einrichtung nach einem der Ansprüche 1 bis 6, **dadurch gekennzeichnet,** daß der Einsatzbehälter (18) mit einem Deckel (18a) versehen ist, der als Gasverteiler gestaltet ist, an den die mindestens eine schlauchartige Leitung (25) für das gasförmige Kältemittel ($K_2$) angeschlossen ist.

8. Einrichtung nach einem der Ansprüche 1 bis 7. **da durch gekennzeichnet,** daß zwischen den SQUIDs (21) und den Gradiometern (24a - 24c) verlaufende elektrische Verbindungsleiter (22) vorgesehen sind, die thermisch an das in den schlauchartigen Leitungen (25) strömende gasförmige Kältemittel ($K_2$) angekoppelt sind.

9. Einrichtung nach Anspruch 8, **dadurch gekennzeichnet,** daß die Verbindungsleiter (22) zumindest als Teil der das gasförmige Kältemittel ($K_2$) führenden schlauchartigen Leitungen (25) gestaltet sind.

10. Einrichtung nach einem der Ansprüche 1 bis 9, **dadurch gekennzeichnet,** daß in dem Innenraum (3) des Dewar-Gefäßes (2) eine Füllung mit Glaskugeln vorgesehen ist.

11. Einrichtung nach einem der Ansprüche 1 bis 10, **dadurch gekennzeichnet,** daß das Dewar-Gefäß (2) in eine Liege (14) für einen zu untersuchenden Patienten (P) so integriert ist, daß die zu detektierende Feldquelle des Patienten (P) oberhalb oder seitlich des Dewar-Gefäßes (2) liegt.

12. Einrichtung nach einem der Ansprüche 1 bis 11, **dadurch gekennzeichnet,** daß das Dewar-Gefäß (2) so bezüglich der Feldquelle angeordnet ist, daß der Hals (4) tiefer als der Innenraum (3) liegt.

13. Einrichtung nach Anspruch 11 oder 12, **dadurch gekennzeichnet,** daß die Patientenliege (14) zur passiven und/oder aktiven Unterdruck-Belüftung des Bereiches des Dewar-Gefäßes (2) ausgestaltet ist.

14. Einrichtung nach einem der Ansprüche 1 bis 13, **dadurch gekennzeichnet,** daß die Achse des Dewar-Gefäßes (2) einen vorbestimmten Winkel mit der Achse des in dem Innenraum (3) angeordneten Einsatzbehälters (18) bildet.

15. Einrichtung nach einem der Ansprüche 1 bis 14, **dadurch gekennzeichnet,** daß das Dewar-Gefäß (2) zumindest weitgehend rotationssymmetrisch aufgebaut ist.

FIG 1

EP 0 361 137 A1

FIG 2

FIG 3

FIG 4

Europäisches
Patentamt

**EUROPÄISCHER RECHERCHENBERICHT**

Nummer der Anmeldung

EP 89 11 6322

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 200 958 (SIEMENS AG) * Zusammenfassung; Figuren 1,2 * --- | 1 | G 01 R 33/035 |
| A | PATENT ABSTRACTS OF JAPAN, Band 10, Nr. 185 (E-441)[2341], 27. September 1986, page 82 E 441; & JP-A-61 104 681 (HITACHI LTD) 22-05-1986 * Zusammenfassung * --- | 1 | |
| A | WO-A-8 504 489 (GEO-SENSORS CORP.) --- | | |
| A | EP-A-0 156 240 (GENERAL ELECTRIC) ----- | | |

| | | | RECHERCHIERTE SACHGEBIETE (Int. Cl.5) |
|---|---|---|---|
| | | | G 01 R 33 |

Der vorliegende Recherchenbericht wurde für alle Patentansprüche erstellt

| Recherchenort | Abschlußdatum der Recherche | Prüfer |
|---|---|---|
| DEN HAAG | 06-12-1989 | HAASBROEK J.N. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.......................................................................
& : Mitglied der gleichen Patentfamilie, übereinstimmendes Dokument

EPO FORM 1503 03.82 (P0403)